(19)

Europäisches
Patentamt
European
Patent Office
Office européen
des brevets

(11) **EP 4 197 506 A1**

(12) **EUROPEAN PATENT APPLICATION**

(43) Date of publication:
**21.06.2023 Bulletin 2023/25**

(21) Application number: **21214289.7**

(22) Date of filing: **14.12.2021**

(51) International Patent Classification (IPC):
***A61F 13/49*** *(2006.01)*   ***A61F 13/15*** *(2006.01)*
***A61F 13/505*** *(2006.01)*

(52) Cooperative Patent Classification (CPC):
**A61F 13/49003; A61F 13/15268; A61F 13/505**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Ontex BV**
  **9255 Buggenhout (BE)**
• **Ontex Group NV**
  **9320 Erembodegem (BE)**

(72) Inventors:
• **COBBAERT, Dries**
  **1770 Liedekerke (BE)**
• **WEBER, Ainas**
  **53474 Bad Neuenahr-Ahrweiler (DE)**
• **IDELSON, Alissa**
  **53359 Rheinbach (DE)**
• **LAMBERTZ, Christina**
  **56566 Neuwied (DE)**

(74) Representative: **Macchetta, Andrea**
  **Ontex BV**
  **Korte Keppestraat 21**
  **9320 Erembodegem-Aalst (BE)**

(54) **RE-USABLE SHELL AND DISPOSABLE INSERT WITH INSERT POSITIONING SYSTEM**

(57)    A disposable absorbent insert comprising: a liquid permeable topsheet; a liquid impermeable backsheet; and an absorbent core comprising absorbent material therein and being sandwiched between said topsheet and backsheet; wherein the insert comprises a perimeter formed by first and second transverse edges and first and second longitudinal edges connecting the first and second transverse edges; a longitudinal centerline extending substantially parallel to said first and second longitudinal edges and interposed therebetween such to divide said insert into a first longitudinal half between said longitudinal centerline and said first longitudinal edge and a second longitudinal half between said longitudinal centerline and said second longitudinal edge; and a transverse centerline extending substantially parallel to said first and second transverse edges and interposed therebetween such to divide said insert into a first transverse half between said transverse centerline and said first transverse edge and a second transverse half between said transverse centerline and said second transverse edge; wherein the absorbent core comprises a core wrap wherein a top layer of the core wrap is adhered to a bottom layer of the core wrap such that one or more channels substantially free of absorbent material are formed, wherein said channels are substantially translucent when viewed in a planar direction from the topsheet-side of said insert and wherein said insert is joinable to a reusable outer shell, and wherein, when joined, a portion and/or element of said outer shell is viewable from said channels.

**FIG. 1A**

EP 4 197 506 A1

**Description**

**TECHNICAL FIELD**

The disclosure relates to absorbent articles such as disposable absorbent articles, preferably selected from the group consisting of diapers (whether for baby or adults), pants (whether for baby or adults), briefs, and combinations thereof generally of the hybrid type i.e. a re-usable and/or washable outer shell and a disposable (generally single-use) absorbent insert cooperable with said outer shell.

**BACKGROUND**

Hybrid-type articles, comprising a re-usable outer shell and disposable absorbent insert, are becoming more and more sought after in the market in view of their sustainable benefits and reduced waste production.

Different designs are available for such products such as described in EP 3 842 017 A1 wherein the outer shell is generally a pant-type component that may comprise elastic components therein and the disposable absorbent insert comprises liquid permeable topsheet, liquid impermeable backsheet and absorbent core sandwiched therebetween and is generally re-fastenably joinable to a crotch region of the outer shell.

Other examples are described in EP 3 659 563 A1 where the disposable insert is re-fastenably joined to the outer shell and the outer shell is in the form of a re-fastenable diaper pant; and EP 3 895 673 A1 wherein the disposable inserts are biodegradable and/or compostable.

Thus, although there have already been significant advancements in this field, there still remains a need to further improve hybrid-type articles so as to provide improved convenience of use such as correct location of the insert onto the outer shell, particularly when a specific front/back orientation is desirable and/or for accurate placement according to the miction point depending on gender (boy/girl) and/or correct placement of channelled core inserts where a specific position (not only front/back orientation) is preferable for optimal functionality of the channel for optimal liquid distribution.

**SUMMARY**

In a first aspect, the disclosure relates to a disposable absorbent insert comprising: a liquid permeable topsheet; a liquid impermeable backsheet; and an absorbent core comprising absorbent material therein and being sandwiched between said topsheet and backsheet; wherein the insert comprises a perimeter formed by first and second transverse edges and first and second longitudinal edges connecting the first and second transverse edges; a longitudinal centerline extending substantially parallel to said first and second longitudinal edges and interposed therebetween such to divide said insert into a first longitudinal half between said longitudinal centerline and said first longitudinal edge and a second longitudinal half between said longitudinal centerline and said second longitudinal edge; and a transverse centerline extending substantially parallel to said first and second transverse edges and interposed therebetween such to divide said insert into a first transverse half between said transverse centerline and said first transverse edge and a second transverse half between said transverse centerline and said second transverse edge; wherein the absorbent core comprises a core wrap wherein a top layer of the core wrap is adhered to a bottom layer of the core wrap such that one or more channels substantially free of absorbent material are formed, wherein said channels are substantially translucent when viewed in a planar direction from the topsheet-side of said insert and wherein said insert is joinable to a re-usable outer shell, and wherein, when joined, a portion and/or element of said outer shell is viewable from said channels.

In a second aspect, the disclosure relates to a re-usable outer shell comprising: a front and back waist region having uppermost and lowermost edges; and a crotch region interposed between the front and back waist region, wherein the uppermost edges of the waist region form a waist opening and the lowermost edges of the waist region together with lateral extremities of the crotch region form two oppositely disposed leg openings; and wherein at least the body-facing surface of the crotch region is adapted for receiving an absorbent insert according to any of the preceding claims; and wherein at least a portion of said crotch region comprises a placement indicia having a shape, when viewed in a planar direction, that substantially corresponds to the shape of said channels, wherein said indicia is viewable through said channels when the insert is joined to outer shell.

In a further aspect, the disclosure related to an absorbent assembly comprising: an insert and an outer shell, preferably wherein the placement indicia has a first colour, the channels have a second colour, and wherein the channels overlaying the placement indicia has a third colour; and wherein the delta E* between the first colour and second colour is at least 0.70, preferably at least 0.75, according to the method herein. Preferably, wherein the channels have a first width and the placement indicia has a second width, and wherein the second with is greater than the first width, preferably wherein the second with is from 20% to 100%, preferably from 25% to 75%, even more preferably from 30% to 60%, greater than the first width.

In a further aspect, the disclosure relates to a kit of parts comprising: a plurality of inserts and one or more outer shells, preferably wherein the plurality of inserts differ in at least one of: size, absorbency, biodegradable and/or compostable content, shape, and number of components contained therein, channel shape, channel size, and channel position.

**BRIEF DESCRIPTION OF THE FIGURES**

**Fig. 1A Is** an illustration, top planar view (topsheet-side), of an insert according to an embodiment herein.

**Fig. 1B** Is an illustration, bottom planar view (backsheet-side), of an insert according to an embodiment herein.

**Fig. 2** Is an illustration, cross-section view, of an insert according to an embodiment herein.

**Fig. 3** Is an illustration, top planar view (body-facing side), of a re-usable outer shell according to an embodiment herein.

**Fig. 4** Is an illustration, top planar view (body-facing side), of a re-usable outer shell according to an embodiment herein.

**Fig. 6** Is an illustration, top planar view (body-facing side), of a re-usable outer shell according to an embodiment herein.

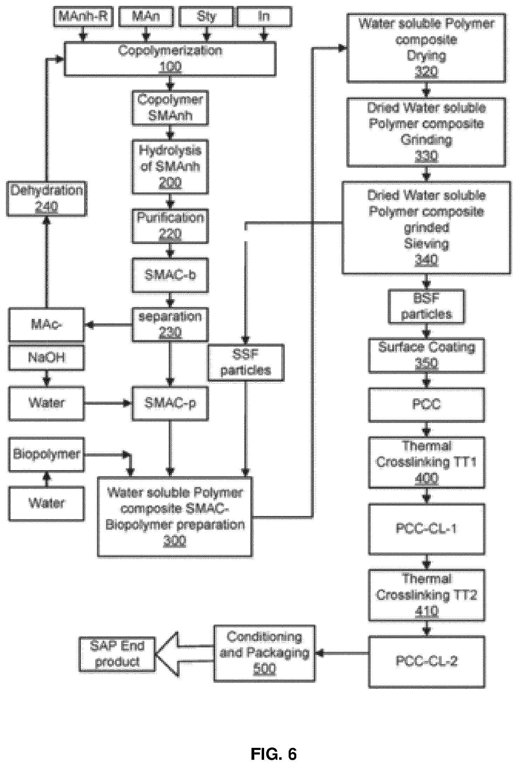

**Fig. 6** Illustrates an exemplary process for the production of high-AUL Bio-SAP.

**DETAILED DESCRIPTION**

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" or "substantially" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" or "%wt" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The terms "nonwoven", nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ.

Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The term "disposable" refers to absorbent articles and/or inserts that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a unitary structure with other elements or as a separate element joined to another element.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

"Color", as used herein, includes any color in the CIELAB color space including primary color, secondary color, tertiary color, the combination thereof, as well as black and white. As used herein "white" is defined as having $L^* > 90$, $-2 < a^* < 2$, and $-2 < b^* < 2$.

CIE $L^*a^*b^*$ ("CIELAB") is the most commonly used color space specified by the International Commission on Illumination (French Commission internationale de l'eclairage, hence its CIE initialism). It describes all the colors visible to the human eye and was created to serve as a device independent model to be used as a reference. The three coordinates of CIELAB represent the lightness of the color ($L^* = 0$ yields black and $L^* = 100$ indicates diffuse white; specular white may be higher), its position between red/magenta and green ($a^*$, negative values indicate green while positive values indicate magenta) and its position between yellow and blue ($b^*$, negative values indicate blue and positive values indicate yellow). The asterisk (*) after L, a and b are part of the full name, since they represent $L^*$, $a^*$ and $b^*$, to distinguish them from Hunter's L, a, and b.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

THE OUTER SHELL

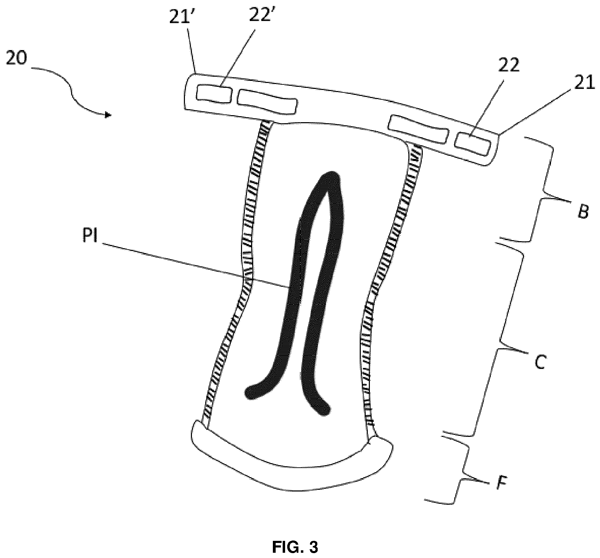

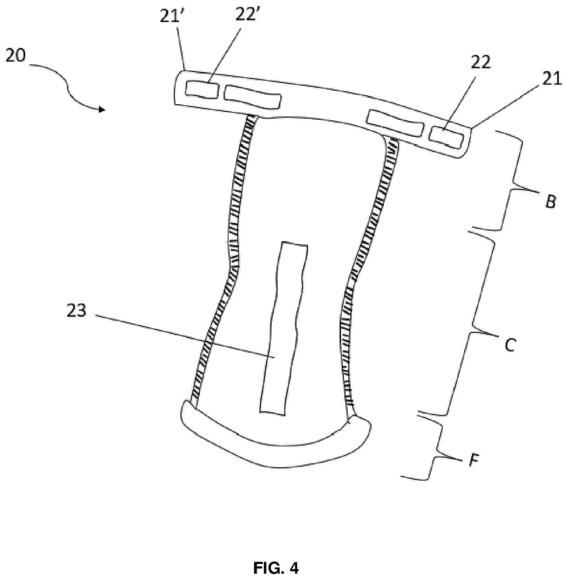

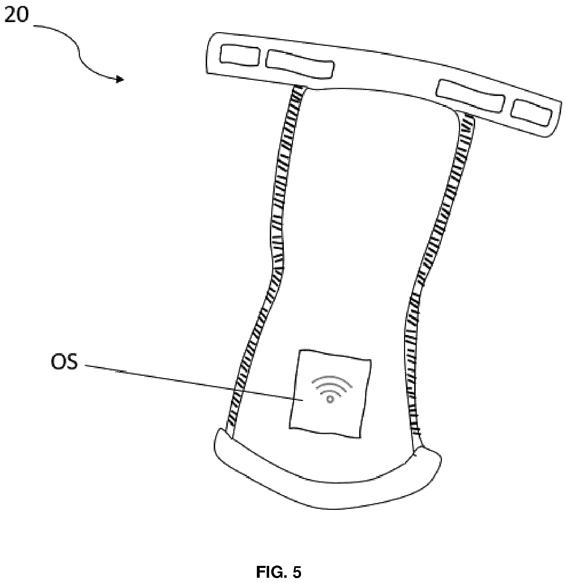

As exemplified in Figs. 3-5, re-usable outer shells (20) herein preferably comprise: a front (F) and back (B) waist region having uppermost and lowermost edges; and a crotch region (C) interposed between the front and back waist region (F, B), wherein the uppermost edges of the waist region form a waist opening and the lowermost edges of the waist region together with lateral extremities of the crotch region (C) form two oppositely disposed leg openings; and wherein at least the body-facing surface of the crotch region (C) is adapted for receiving an absorbent insert (1) as described herein. At least a portion of the crotch region (C) comprises a placement indicia (PI) having a shape, when viewed in a planar direction (e.g. viewed from the skin/body-facing side over a plane formed by the longitudinal and transverse axis and looking downward in a direction substantially parallel to a thickness direction), that substantially corresponds to the shape of said channels (13), wherein said indicia (PI) is viewable through said channels (13) when the insert (1) is joined to outer shell (20). Advantageously this allows for easy and effective location of the insert correctly onto the outer shell.

In an embodiment, the placement indicia (PI) comprises, preferably consists of, a stiffening element having a higher resistance to bending compared to any other position and/or element of the outer shell (20) being proximally inboard and/or outboard of said indicia (PI). This allows for limiting risks of distortions that may impact visibility of the indicia impacting correct placement of the insert as well as at the same time improve cup formation and support sagging especially when the insert is wetted and/or loaded with liquid.

Preferably, the placement indicia (PI) comprises a first graphic and/or colour that is viewable through the channels (13) when the insert (1) is joined to the outer shell (20). Advantageously this allows for a simple identification system enabled by functional aspects of the article.

Preferably, the placement indicia (PI) has a first colour and wherein the channels (13) have a second colour, and wherein the delta E* between the indicia (PI) and the channels (13) is at least 0.4, preferably at least 0.7, according to the method herein. Advantageously this permits to ensure a visible change between correctly or incorrectly placed insert simply by viewing the colour change through the channels during and/or after the joining step.

In an embodiment, at least a portion of said crotch region (C) corresponding with a wetness indicator (10) of the insert (1) is translucent such that said wetness indicator is viewable from a garment facing side of said re-usable outer shell (20) or comprises an optical sensor (OS) adapted to automatically detect a colour change of said wetness indicator (10) and send a corresponding signal to an application device. Advantageously this not only allows to provide effective saturation indication when using a hybrid-article but further allows for at the same time providing indicia to correctly position the insert in the right front/back relationship that may be particularly useful when designing inserts that have a non-uniform absorption profile (e.g. varying basis weight of absorbent material for optimised absorption and limited over-specked or wasted material which then require the correct front/back orientation on placement). In the latter case the insert may be correctly placed when the wetness indicator is fully/entirely viewable through the outer shell (when viewed on the garment side thereof). This may be verified either directly, i.e. visually, by a caregiver viewing the wetness indicator indicia or indirectly, by automatic identification by the sensor that may then provide a warning via an application device to a caregiver accordingly.

The re-usable outer shell (20) may comprise re-fastenable or permanent side seams joining the front and back waist regions, preferably in the form of a pant; or the back waist region comprises transversely extending side panels each comprising a fastener for coupling to a landing zone positioned at a garment facing side of the front waist region, preferably in the form of a diaper.

In an embodiment the crotch region of the re-usable outer shell (20) comprises a window (23) for viewing the wetness indicator (10) and/or optical sensor from a garment-facing side of said shell (20). The crotch region is generally adapted for receiving an absorbent insert (1) as described herein and wherein at least a portion and/or area of said crotch region comprises said window (23) that substantially corresponds with the position of the wetness indicator (10), when the insert (1) is joined to the shell (20). Advantageously this allows for visual inspection of saturation and/or correct positioning directly by a subject on use.

The window herein may be in the form of a cut-out or opening, or may comprise a translucent and/or transparent material such as a film.

In an embodiment, the optical sensor (OS) is comprised by a detection device further comprising one or more light sources, preferably a plurality of light sources having at least a first colour and a second colour, wherein the first colour is blue and the second colour is red, preferably said light sources consisting of LEDs, more preferably said light sources being positioned at a distance from each other and from the optical sensor; even more preferably the detection device further comprising a battery and a transmitter and being detachably connected to said outer shell.

Optical sensors and devices suitable herein may be as described in co-pending application EP 3 888 606 A1.

In a preferred embodiment, the sensor and/or detection device is positioned on a body-facing surface of the re-usable shell (20) with the optical sensor (OS) facing upwards towards a body-facing side such to come into, preferably direct, contact with the backsheet of the insert comprising the wetness indicator. Alternatively, the sensor and/or detection device may be positioned on a garment-facing surface of the re-usable shell at a position corresponding to the window so that the optical sensor is in direct or indirect, preferably indirect, contact with the backsheet of the insert comprising the wetness indicator via the window described herein.


THE INSERT


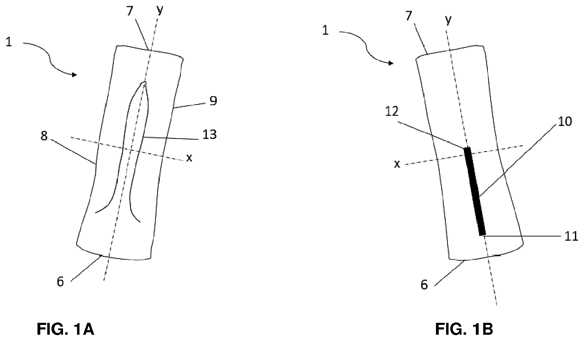

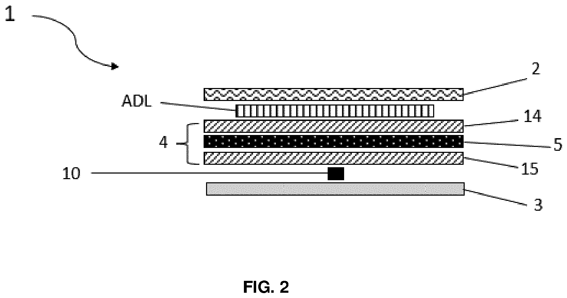

As exemplified in Figs. 1A, 1B, and 2, inserts herein typically comprise a liquid permeable topsheet (2), a liquid impermeable backsheet (3); and an absorbent core (4) comprising absorbent material (5) therein and being sandwiched between said topsheet (2) and backsheet (3).

Inserts herein may further comprise an acquisition distribution layer (ADL) positioned between the topsheet and the absorbent core, preferably between the topsheet and the top core wrap layer. The acquisition distribution layer may have a basis weight of from 15 gsm to 55 gsm, preferably from 18 gsm to 50 gsm, even more preferably from 19 gsm to 45 gsm. Preferably the acquisition distribution layer is selected from a carded air-through bonded nonwoven, a carded thermobonded calendered nonwoven, a spunbond nonwoven, and combinations thereof.

Generally, the insert (1) comprises a perimeter formed by first and second transverse edges (6, 7) and first and second longitudinal edges (8, 9) connecting the first and second transverse edges (6, 7); a longitudinal centerline extending substantially parallel to said first and second longitudinal edges (8, 9) and interposed therebetween such to divide said insert (1) into a first longitudinal half between said longitudinal centerline and said first longitudinal edge (8) and a second longitudinal half between said longitudinal centerline and said second longitudinal edge (9); and a transverse centerline extending substantially parallel to said first and second transverse edges (6, 7) and interposed therebetween such to divide said insert (1) into a first tranverse half between said transverse centerline and said first transverse edge (6) and a second transverse half between said transverse centerline and said second transverse edge (7).

Inserts herein preferably comprise a pair of barrier cuffs extending along the first and second longitudinal edges (8, 9) and arranged to provide lateral barriers preventing exudate leakage. Typically, the barrier cuffs comprising a hydrophobic nonwoven and one or more elastics at an apex position thereof such to form standing gathers when the insert is extended for placement within the re-usable outer shell.

Generally, inserts herein are free of transversely extending side panels such as elastic side panels.

Backsheets for use herein are as commonly known in the art and may be breathable and/or comprise a laminate of a film, preferably polyethylene (PE) based or bio-PE based, and a nonwoven layer with the nonwoven layer being positioned on the outermost surface at a garment facing side thereof.

The absorbent core (4) comprises one or more attachment zones wherein a top layer of a core wrap (14) is adhered to a lower layer (also herein "bottom" layer) of a core wrap (15) such that one or more channels (13) substantially free of absorbent material are formed, preferably wherein the wetness indicator (10), when present, extends between said channels and preferably overlaps at least a portion of said channels. It is understood herein that the core wrap may be a single layer that is folded to envelope the absorbent material therein and having top and bottom layers as described above, or may be comprised of two separate layers forming the top and bottom core wrap layers described above.

The absorbent material is generally enclosed by the core wrap and the channels (13) are typically surrounded by absorbent material said absorbent material generally demarcating the profile of the channels (13), at least when viewed in a planar direction. Absorbent material is thus typically extending inboard and/or outboard of the channels (13) when viewed in a planar direction. The channels herein thus preferably do not extend all the way to the perimeter of the absorbent core (4) or any of the transverse edges (6, 7) and longitudinal edges (8, 9) of the absorbent core (4).

In a preferred embodiment, channels (13) herein are longitudinally extending and cross both the transverse centerline (x) at a first crossing point and the longitudinal centerline (y) at a second crossing point, wherein the first and second crossing points are different and preferably wherein the second crossing point is positioned closer to the second transverse edge (7) than the first crossing point, and preferably wherein the second transverse edge (7) is positioned proximal to the back region (B) of the outer shell (20) when said insert (1) is connected thereto. Advantageously this allows for improved cup formation in a hybrid concept as well as retaining liquid distribution advantages by guiding exudates to the back of the core.

The core wrap layer may be a single layer or multilayer and comprises, preferably consists of a nonwoven. Preferably, the nonwoven is selected from spunbond nonwoven, meltblown nonwoven, carded nonwoven and combinations thereof. Preferably the core wrap is multilayer comprising spunbond and meltblown such as spunbond-meltblown-spunbond nonwovens (SMS).

Preferably, the core wrap has a basis weight of less than 30 gsm, preferably less than 25 gsm, even more preferably from 5 gsm to 15 gsm, even more preferably from 6 to 10 gsm. Advantageously such low basis weights aid to attain a sufficiently translucent channel when top/bottom core wrap layers are bonded together typically via adhesive and/or mechanical bonding (such as ultrasonic bonding or heat pressure bonding).

The channels (13) are substantially translucent when viewed in a planar direction from the topsheet-side of said insert (1) and the insert (1) is joinable to a re-usable outer shell (20), and when joined a portion and/or element of said outer shell (20) is viewable through said channels. Advantageously this allows for correct location of placement of the insert, especially since absorbent material has been found to result in very high opacity index and low translucency that otherwise does not permit a user to see what is beneath and hence being a bottleneck to accurate placement of the insert.

Preferably, the re-usable outer shell (20) as will be described in more detail herein comprises placement indicia (PI) having a shape that substantially corresponds to the shape of the channels (13) when viewed in a planar direction. Advantageously this allows not only for correct placement front-to-back of the insert but further optimal channel positioning for optimal liquid distribution by relative positioning vs miction point (e.g. based on sex male/female).

In an embodiment, the channels have a first opacity and areas of the core inboard and/or outboard of the channels a second opacity wherein the first opacity is less than the second opacity, according to the method herein.

Preferably, the first opacity is less than 60%, preferably less than 50%, even more preferably less than 45%, even more preferably less than 40%, even more preferably from 5% to 35%. Advantageously, a sufficiently low opacity allows for correct viewing of the placement indicia through the channel.

Inserts (1) herein preferably comprise a wetness indicator (10) that is viewable from a garment-facing side of the backsheet (3) and the insert (1) is joinable to a re-usable outer shell (20) preferably such that the wetness indicator (10) is viewable from a garment-facing side of the re-usable outer shell (20).

The inserts herein may be substantially rectangular in shape but may equally be shaped such as hourglass and/or substantially T-shaped.

In an embodiment, the absorbent material comprises, preferably consists of, superabsorbent particles and/or cellulose fibers.

The absorbent cores herein may comprise at least 60%wt of superabsorbent particles, in particular at least 70%wt, preferably at least 80%wt, preferably at least 90%wt, by total weight of the core.

In an embodiment, the superabsorbent particles comprise a blend of superabsorbent particles comprising a first superabsorbent particles (SAP1) and a second superabsorbent particles (SAP2), wherein the first superabsorbent particles (SAP1) have an AUL that is greater than the AUL of the second superabsorbent particles (SAP2), and wherein the first superabsorbent particles (SAP1) have an AUL of greater than 15 g/g, according to the test method herein, preferably wherein the first superabsorbent particles (SAP1) have a particle size distribution that is greater than that of the second superabsorbent particles (SAP2). Preferably wherein at least the second superabsorbent particles (SAP2) comprises, preferably consists of, bio-based superabsorbent composite polymer particles comprising a synthetic hydrophobic polymer and a natural biopolymer, more preferably composed of a composite polymer comprising styrene maleic acid copolymer and a biopolymer of animal or vegetal origin; or non-composite polymer particles selected from polyacrylic acid, sodium salt, crosslinked, partly neutralized superabsorbent particles.

Preferably the second superabsorbent particles (SAP2) comprise, preferably consist of, Low-AUL Bio-SAP. "Low-AUL Bio-SAPs" means that they generally have AUL (as measured according to the test method herein) of less than 20 g/g, typically less than 15 g/g, and typically belong to the following classes: (a) materials consisting only of crosslinked biopolymers; (b) materials consisting only of crosslinked synthetic polymers; (c) composite materials consisting of synthetic polymers and biopolymers in certain variations: in inter crosslinking type connection with or without chemical agents; graft type; intercomplexate type and interpenetrate type. Copolymers such as styrene maleic acid are typically used in copolymerization processes for achieving Low-AUL Bio-SAP. Other exemplary sources include alpha-1,3 glucan, starch, starch and/or sodium salts, sugar and derivatives. Exemplary commercially available Low-AUL Bio-SAPs for use herein include: SAPs derived from charged-modified starches as sold from TETHIS 5237 Capital Blvd.; Raleigh, NC 27616, USA and further exemplified in US20200054782 A1 herein incorporated by reference; SAPs comprising a 100% acrylic acid co-acrylamide with little to no crosslink shell, and the like all generally having AUL of less than 20 g/g according to the test method herein.

Preferably, the first superabsorbent particles (SAP1) are free of Low-AUL Bio-SAP. Advantageously, this limits rewet drawbacks as described herein.

In an embodiment, the SAP1 is a High-AUL Bio-SAP "composite polymer" (i.e. a biodegradable superabsorbent composite polymer having an AUL of greater than 15 g/g, preferably greater than 20 g/g, as will be described in more detail herein below). The composite polymer may comprise a synthetic hydrophobic polymer and a natural biopolymer. More specifically, the composite polymer may comprise styrene maleic acid copolymer and a biopolymer of animal or vegetal origin in conformity with the technological flow chart presented in Fig. 6. Styrene maleic acid copolymer is preferably in salt form, more preferably in the form of monovalent cation salt. Alternatively, the High-AUL Bio-SAP, although less preferred, may comprise "non-composite polymers" and rather be selected from certified biomass superabsorbent polymers having AUL of greater than 15 g/g, preferably greater than 20 g/g, and typically certified by REDcert2 (https://www.redcert.org/images/SP_RC%C2%B2_Biomass-balanced_products_V1.0.pdf ). An example may be a polyacrylic acid, sodium salt, crosslinked, partly neutralized SAP from up to 100% allocated biomass feedstock such as commercially available HySorb® B 6600MB manufactured and sold by BASF SE, Ludwigshafen, Germany.

In an embodiment of the High-AUL Bio-SAP "composite polymer", the styrene moiety in the synthetic polymer can be replaced by other hydrophobic moieties. Exemplary synthetic polymers are: poly(maleic anhydride-co- methyl vinyl ether) (Gantrez), poly(vinyl chloride-co-maleic acid) and poly[(maleic anhydride)- alt-(vinyl acetate).

The term "composite" as herein used refers to a polymeric substance that a) is formed from at least two polymers with different macromolecular chemical structure; and b) the resulting composite is a unique entity that does not separate spontaneously to its components during application. It is understood that the term "composite" may include other substances such as drugs, stimulators, inhibitors, odorants, emollients, plasticizer and others.

The term "anionic" refers to a polymeric composite generating in aqueous media a negative electrochemical potential as the result of the presence in its structure of some free acid functional groups capable of dissociating into anions.

In an embodiment described in Fig. 6, the production process starts with the synthesis of copolymer (styrene-alt-maleic anhydride) by bulk co-polymerization using an excess of about 60-90% of maleic anhydride relative to styrene, whereas maleic anhydride works also as a solvent (operation 100).

Copolymerization is typically executed in Sigma-type mixer machines named Hermetic machines in order to be able to work at high pressures e.g. not exceeding 10 bar or in vacuum conditions (less than IOmbar) with double mantle as well with arms equipped with a heating - cooling system.

The copolymerization process for the production of a superabsorbent polymer typically uses styrene monomer stabilized with organic compounds that inhibit the process of homopolymerization during storage and transportation. Such inhibitors are for example substances such as : amino derivatives, thiols derivatives, and hydroxyl derivates as for example (2-hydroxypropyl)-ethylene diamine compounds, 4-tert-butylcatechol and others) being preferred 4-tert-butylcatechol in proportion of 0.002- 0.008% to the monomer, more preferably is 0.003- 0.007% to the styrene and most preferably 0.004-0.006% to the styrene and the molar fraction of styrene in the reaction mass is 0.05-0.08, preferably 0.1-0.15 and more preferably 0.18-0.21.

The copolymerization may also contain maleic anhydride that is either fresh maleic anhydride MAnh or recovered maleic anhydride MAnh-R that is recycled from a previous batch as schematically showed in Fig. 6. and the amount of fresh maleic anhydride MAnh relative to recovered maleic anhydride MAnh-R is about 10 - 40% (dry basis).

For the copolymerization, further customary agents may be used such as peroxides, azo compounds etc., which form free radicals by thermal decomposition, while the quantity of initiator is 0.05- 0.15 %, preferably 0.07-0.009% and most preferably 0.08-0.12% to a double quantity of styrene adopted for copolymerization.

Next, it may follow the conversion of styrene-alt maleic anhydride copolymer to styrene-alt maleic acid copolymer by hydrolysis with water (process 200) which is done in the same equipment where copolymerization has been made. Conversion of styrene-alt maleic anhydride copolymer to styrene-alt maleic acid copolymer by hydrolysis using a quantity of water higher than the one stoichiometrical required for total hydrolysis (Ws) with an excess which represents 2-3% versus to the stoichiometrical value, preferably in excess of 4-5% to the stoichiometrical water and most preferably 5-10% in excess to the stoichiometrical water.

The total quantity of water necessary to styrene and maleic anhydride copolymer's hydrolysis is typically inserted in the mass of reaction in two steps from which 50% is in the form of 0.005N hydrochloric acid solution and the rest as non-acidulated water.

The acidulated water is typically inserted into the reaction mass in two portions at a mass reaction's temperature that not exceeding 60°C at 15 minutes intervals between each dosage, and the quantity of non-acidulated water is inserted into the mass of reaction also in two portions, from which the first is after 60 minutes from the last portion of acidulated water, then is inserted the last portion of non-acidulated water and mixing of reaction mass for 45-60 minutes in cooling conditions of 35-40°C. Reaction mass resulted after hydrolysis is a wet solid in form of a powdery mass of white colour with a value of bulk density of 0.6-0.8 g/cm3. Further, mass of reaction that resulted after copolymerization and hydrolysis (which is a blend of styrene maleic acid copolymer - SMAC and free maleic acid -MAC, which contains traces amounts of non-reacted styrene and stabilizer for styrene as well as traces of hydrochloric acid) is transferred to perform the purification process of styrene maleic acid copolymer (process 220).

The purification process which represents the extraction of free maleic acid fraction from SMAC polymer mass is typically done in an equipment such as tank-type with mixing in which over reaction mass resulted after hydrolysis of synthetic copolymer is added a quantity of deionized water for purification [Wp] that represents a quantity correlated to the wet mass of reactions (RM) in accordance with the relationship Wp= 2 * RM or Wp= 5 * RM, preferably Wp= 3 * RM.

Purification generally consists of 3-5 extraction stages followed each time by filtration. The number of extraction and filtration operations are established so that the content of the free carboxylic groups found in polymer of SMAC to be between 0.00909-0.0095 mole/gram, preferably between 0.0091-0.0094 mole/gram and most preferably between 0.0092- 0.0093 mole/gram.

The extraction in fact preferably occurs at temperature of 60°C for 30 minutes and each filtration (process 230) is done with known equipment as press filter or Nuce filter. All solutions resulted from filtering are collected into a tank of supernatant in order to process the maleic acid which it contains.

The processing of maleic acid water solution to recovery of maleic anhydride preferably consists of the following operations: a) concentration of maleic acid solution through reverse osmosis; b) spray drying of maleic acid concentrated solution when resulted maleic acid powder and water; c) conversion of maleic acid powder to maleic anhydride by thermal-vacuum dehydration (with technological parameters modified than those mentioned in US Pat No.4,414,398 when in the end is obtained a material called recovered maleic anhydride (MAnh-R). The purified filtrate of SMAC polymer is typically collected in an equipment as Sigma mixer type in order to be processed with biopolymers to obtain the water-soluble composite polymer containing synthetic SMAC polymer and biopolymer [WSPC] (process 300).

The preparation process of polymeric composite [WSPC] containing SMAC and a biopolymer of animal or vegetal origin is preferably preceded by other operations as follows: a) Preparation of a base solution is obtained by dissolution of a solid hydroxide compound in water to 40% by weight, whereas examples of preferred base hydroxide compounds are sodium hydroxide, lithium hydroxide, potassium hydroxide, ammonium hydroxide, preferably sodium hydroxide; b) transformation of styrene maleic acid copolymer obtained in step 230 into styrene-maleic acid monovalent cation salt by neutralization with base solution prepared in a) above in order to obtain a solution of copolymer salt with concentration higher than 25% preferably higher than 35 % and most preferably higher than 50%. Neutralization of the SMAC copolymer is 48-58%, preferably 50-56% and most preferably 52-54%; c) preparation of the biopolymer (as gelatin, albumin, casein, soy, guar or starch, preferably is gelatin) as water solution of 40% by weight in water; d) preparation of polymeric composite is done by treating the solution of styrene maleic acid salt with biopolymer solution at temperature of 55-75°C for 30 minutes.

The amount of biopolymer relative to copolymer in the composite is preferably from 4-6 % (dry basis), preferably 8-10 % (dry basis) and most preferably 12-14 % (dry basis).

The blending of the composite mass typically continues during 4-5 hours until the polymeric mass is transformed from the viscous solution into a partially dried granular mass with a moisture content not higher than 20%. This partially dried polymeric composite material WSPC in granular form is typically subjected to a supplementary drying (process 320) at temperatures of preferably 75-85°C on conveyor belt type or Rotary type in order to obtain final drying of until the moisture content is less than 14%, preferably less than 12% and most preferably less than 8%.

Further steps of drying, grinding and sieving are preferably carried out (process 330 + process 340) to obtain two types of solid phases called herein large solid phase (LSP) with granulometric distribution higher than 100 microns, preferably of 100 -850 microns that corresponds to be used in the manufacture of diapers and a small solid phase (SSF) with granulometric distribution up to 100 microns. The small solid phase SSF is re-used in the preparation of the next new batch of SAP that comes into the process 300.

The large solid phase LSP may be exposed to post-treatment surface coating processes using known chemical substances as: glycerin ethylene glycol, propylene glycol or polyether hydroxyl with properties of biodegradability. Examples of preferred coating materials are hydroxyl polyether, more preferably polyethylene glycol - PEG 200 used at a rate of 0.2-2% by weight (dry basis) to LSP, preferably at a rate of 0.5-1.5% by weight and most preferably at a rate of 0.8-1.2% by weight to LSP.

Surface coating may be applied using equipment like powder coating machines at temperatures of generally 30-70°C, preferably at temperatures of 35-65°C and most preferably at temperatures of 40-60°C for 30 -90 minutes, preferably for 40-75 minutes and most preferably for 50-60 minutes. As a result of this process is resulted the material called Polymer Composite Coated Treated- PCC (operation 350).

The obtained material after surface coating (Polymer Composite Coated) may be subdue to a thermal treatment called first thermal treatment (TT1) (operation 400) consisting in warming of particles mass in hot air with temperature of 90-140°C for 30-150 minutes, preferably with temperatures of 100-135°C for 45-120 minutes and most preferably by bulk thermal crosslinking at temperatures of 110-120°C, for 60-90 minute using equipment of conveyor belt type or shaking rotary type when is resulted an intermediary material called Polymer Composite Coated first crosslinked (PCC-CL-I).

The material PCC-CL-I may be subdue to a new thermal treatment (TT2) (operation 410) in hot air with temperature of 120-150°C for 5-30 minutes, preferably at temperatures of 125-145°C for 10-25 minutes and most preferably to temperature of 120-140°C for 15-20 minute in the same type of equipment used for TT1 and is obtained the Polymer Composite Coated second crosslinked (PCC-CL-2). The material (PCC-CL-2) may then be conditioned for 24 hours in an atmosphere in which the air has a moisture content of 65% and temperature of 20°C and then is packaged in sealed polyethylene bags (operation 500).

The material resulting after conditioning is a biodegradable SAP with AUL higher than 20g/g in aqueous solution of 0.9% NaCl at pressure of 0.9 psi, which represents the end product of the manufacturing process which is the object of the present invention, i.e. the "High-AUL biodegradable superabsorbent composite polymer" referred to herein.

Reference is made to co-pending application EP21152698.3 for further examples of Low-AUL Bio-SAP and High-AUL Bio-SAPs that may be used in absorbent cores herein, with particular reference to Example 1.

In an embodiment, the first superabsorbent particles (SAP1) are comprised at a level of less than 80%wt, preferably from 10%wt to 32%wt; and the second superabsorbent particles (SAP2) are comprised at a level of at least 20%wt, preferably from 25%wt to 100%wt, more preferably from 30% to 90%, even more preferably from 35% to 80%, even more preferably from 40% to 70%, even more preferably from 45% to 68%, by total weight of superabsorbent particles.

Preferably, the AUL ratio AULSAP1 /AULSAP2) of the first superabsorbent particles (SAP1) and second superabsorbent particles (SAP2) is greater than 1.4, preferably greater than 1.5, more preferably from 1.6 to 5, even more preferably from 1.7 to 3. Advantageously, this allows to ensure the right balance of reduced rewet and fast liquid absorption especially on the lower layer and optimal performance under load.

In an embodiment, the first superabsorbent particles (SAP1) have a lower absorption speed (typically according to the vortex method herein) than the second superabsorbent particles (SAP2). Preferably, the vortex time (according to the vortex method herein) of SAP1 is more than 50 seconds, preferably from 60 seconds to 90 seconds. Preferably, the absorption speed of SAP2 is less than 45 seconds, preferably less than 40 seconds, even more preferably from 15 seconds to 35 seconds.

The absorbent inserts herein preferably comprise a wetness indicator (10) which is visible from the exterior of the insert and/or article and which changes appearance when contacted with body exudates, in particular urine. The wetness indicator (10) may be placed, when seen from the exterior of the insert and/or article, between the two channel-forming areas and/or channels according to some embodiments herein.

The wetness indicator serves the role of alerting the wearer that the insert has been soiled and may need changing.

In an embodiment, the absorbent core () comprises a core wrap enclosing the absorbent material therein, and typically wherein (when present) the wetness indicator (10) is positioned between a garment-facing side of the core wrap and a body-facing surface of the backsheet (3).

THE ABSORBENT ASSEMBLY

Absorbent assemblies herein may comprise an insert (1) as described herein coupled to an outer shell (20) as described herein.

Preferably, the placement indicia (PI) has a first colour and wherein the channels (13) have a second colour, and wherein the delta E* between the indicia (PI) and the channels (13) is at least 0.45, preferably at least 0.70, even more preferably at least 0.75, preferably at least 0.85, preferably at least 0.90, preferably at least 1.0, or at least 2.5, or at least 3.0, or at least 4.0, or at least 5.0, or at least 10 or at least 15, according to the method herein. The difference in colour may be obtained by using different pigmentation for the fibers and/or film of the core wrap layer and the backsheet layer.

In a highly preferred embodiment, the placement indicia (PI) has a first colour, the channels (13) have a second colour, and wherein the channels when overlaying the placement indicia have a third colour; and wherein the delta E* between the second colour and third colour is from greater than 0 to 2.0, preferably from 0.1 to 1.5, even more preferably from 0.2 to 1.0, even more preferably from 0.3 to 0.85, even more preferably from 0.4 to 0.70, according to the method herein. Whilst it is desirable that the delta E* is as large as possible between the placement indicia (PI) and the channels (13) to make a pronounced visual difference aiding insert location, it may be advantageous for the delta E* between the channels (when seen alone and not overlaying the placement indicia) and the channels when joined and/or overlaying the placement indicia to be rather smaller so as to allow the user to substantially visually recognise the placement indicia lying beneath.

It has been found that a delta E* of 0.7 is already sufficient to obtain bond areas which are visually distinct from the unbonded areas (i.e. when insert channel is correctly joined to the outer shell and overlaying the indicia; or when the channel is not so correctly joined or unjoined), such that the visual difference is noticeable to the naked eye. However, as delta E* increases, the respective visual difference becomes more distinguishable and more pronounced aiding visual indication for correct placement of the insert.

Further colour distinctions can be found using delta C* and delta H*. It has been found that a sufficient and significant colour distinction can be achieved by providing a multilayered structure which may have delta C* of at least 1, or at least 3 or at least 5 between the bonded areas and the unbonded areas (i.e. between channels and channels overlaying the placement indicia). Also, sufficient and significant colour distinction can be achieved by providing a multilayered structure which may have delta H* of at least 1, or at least 1.2, or at least 1.5, or at least 2 between the bonded areas and the unbonded areas.

The channels (13) herein described preferably have a first width extending substantially perpendicular to the longest dimension thereof (and generally extending substantially perpendicular to a longitudinal centerline of the insert). It is nevertheless understood that in case of curved channels or channels that also cross the longitudinal centerline from one have to the other half (e.g. left half to right half and vice versa) the width of the channel in such transverse portion may correspond to the dimension being substantially parallel to the longitudinal centerline of the insert.

The placement indicia (PI) herein described preferably has a second width, and wherein the second with is greater than the first width, preferably wherein the second with is from 20% to 100%, preferably from 25% to 75%, even more preferably from 30% to 60%, greater than the first width. Advantageously this allows for accurate location of multiple inserts having different channel sizes and/or absorbency levels e.g. day and night.

There may be provided a kit of parts comprising: a plurality of inserts (1) as described herein; and one or more outer shells (20) as described herein. Such kits may allow for extended use of the re-usable outer shells.

Preferably, the plurality of inserts (1) differ in at least one of: size, absorbency, biodegradable and/or compostable content, shape, and number of components contained therein, channel shape, channel size, and channel position. Advantageously this allows for different inserts targeting different types of usage e.g. day vs night, size increases of subjects/wearers etc. whilst retaining substantially the same outer shell.

Preferably, the kits herein further comprising a detection device comprising an optical sensor. Advantageously such allowing the use of one detection device for a plurality of inserts that may be different in at least one of: size, absorbency, biodegradable and/or compostable content, shape, and number of components contained therein as described herein above.

TEST METHODS

AUL (Absorbency Under Load, 0.7 psi)

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0.7 psi is a Plexiglas cylinder 60 mm in internal diameter and 50 mm in height. Adhesively attached to its underside is a stainless steel sieve bottom having a mesh size of 36 $\mu$m. The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as W0. Then 0.900 +/- 0.005 g of water-absorbing polymer or material (particle size distribution 150 - 800 $\mu$m or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wa. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0.9% by weight sodium chloride solution is introduced for the surface of the liquid to be level with the filter plate surface without the surface of the filter plate being wetted. A round filter paper 90 mm in diameter and < 20 $\mu$m in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as Wb.

Absorbency under load (AUL) is calculated as follows:

$$AUL\,0.7\,psig/g = Wb - Wa/Wa - W0$$

AUL 0.3 psi and 0.5 psi are measured similarly at the appropriate lower pressure.

Absorption speed (Vortex) measurement:

The vortex test measures the amount of time in seconds required for 2 grams of a superabsorbent material to close a vortex created by stirring 50 milliliters of saline solution at 600 revolutions per minute on a magnetic stir plate. The time it takes for the vortex to close (that the surface of the fluid becomes flat meaning that in the beginning, the centrifugal force that is caused by the rotation of the fluid creates a "coning-in" in the surface, but when the gelling of the SAP proceeds the viscosity of the fluid increases so that the depth of the indentation decreases until it's finally substantially flat) is an indication of the free swell absorbing rate of the superabsorbent material.

Equipment and materials:

1. Beaker, 100 ml.
2. Programmable magnetic stir plate, capable of providing 600 revolutions per minute.
3. Magnetic stir bar without rings, 7.9 mm $\times$ 32 mm, Teflon covered.
4. Stopwatch.
5. Balance, accurate to $\pm$ 0.01 g.
6. Saline solution, 0.9%.
7. Weighing paper.
8. Room with standard condition atmosphere: Temp = 23°C $\pm$ 1°C and Relative

$$Humidity = 50\% \pm 2\%.$$

Test procedure:

1. Measure 50 g $\pm$ 0.01 g of saline solution into the 100 ml beaker.
2. Place the magnetic stir bar into the beaker.
3. Program the magnetic stir plate to 600 revolutions per minute.
4. Place the beaker on the center of the magnetic stir plate such that the magnetic stir bar is activated. The bottom of the vortex should be near the top of the stir bar.

5. Weigh out 2 g ± 0.01 g of the superabsorbent material to be tested on weighing paper.

6. While the saline solution is being stirred, pour the superabsorbent material to be tested into the saline solution and start the stopwatch. The superabsorbent material to be tested should be added to the saline solution between the center of the vortex and the side of the beaker.

7. Stop the stopwatch when the surface of the saline solution becomes flat, and record the time.

8. The time, recorded in seconds, is reported as the Vortex Time.

Opacity Measurement Method

The opacity of a material is the degree to which light is blocked by that material. A higher opacity value indicates a higher degree of light block by the material. Opacity may be measured using a 0.deg. illumination / 45.deg. detection, circumferential optical geometry, spectrophotometer with a computer interface such as the HunterLab LabScan XE running Universal Software (available from Hunter Associates Laboratory Inc., Reston, VA). Instrument calibration and measurements are made using the standard white and black calibration plates provided by the vendor. All testing is performed in a room maintained at about 23°C ± 2°C and about 50% ± 2% relative humidity. Configure the spectrophotometer for the XYZ colour scale, D65 illuminant, 10.deg. standard observer, with UV filter set to nominal. Standardize the instrument according to the manufacturer's procedures using the 1.20 inch port size and 1.00 inch area view. After calibration, set the software to the Y opacity procedure.

To obtain the specimen, a section of the channel (top and bottom core wrap layers in bonded state) may be cut and directly inspected. Cut a piece 10 mm by 50 mm. Precondition samples at about 23°C ± 2°C and about 50% ± 2% relative humidity for 2 hours prior to testing.

Place the specimen over the measurement port. Cover the specimen with the white standard plate. Take a reading, then remove the white tile and replace it with black standard tile without moving the specimen. Obtain a second reading, and calculate the opacity as follows:

$$Opacity = Y \ value \ [(black \ backing) \ / \ Y \ value[(white \ backing) \ x \ 100$$

A total of five substantially identical samples are analyzed and their opacity results recorded. Calculate and report the average opacity and standard deviation for the web measurements to the nearest 0.01%.

Measurement of delta E*

The colour difference measurement is based on the CIE L* a* b* colour system (CIELAB). A flat bed scanner capable of scanning a minimum of 24 bit colour at 1200 dpi and has manual control of colour management (a suitable scanner is an Epson Perfection V750 Pro from Epson America Inc., Long Beach CA) may be used to acquire images. The scanner is calibrated against a colour reflection target compliant to ANSI method IT8.7/2-1993 using colour management software (a suitable package is MonacoEZColour available from X-Rite Grand Rapids, MI) to construct a scanner profile. The resulting calibrated scanner profile is opened within an imaging program that supports sampling in CIE L* a* b* (a suitable program is Photoshop S4 available from Adobe Systems Inc., San Jose, CA) to measure the areas.

Turn on the scanner for 30 minutes prior to calibration. Place the IT8 target face down onto the scanner glass and close the scanner lid. Open the MonacoEZColour software and select acquire image using the Twain software included with the scanner. Within the Twain software deselect the unsharp mask setting and any automatic colour correction or colour management options that may be included in the software. If the automatic colour management cannot be disabled, the scanner may not be appropriate for this application. Acquire a preview scan at 200 dpi and 24 bit colour. Insure that the scanned image is straight and first outer surface facing side-up. Crop the image to the edge of the target, excluding all white space around the target, and acquire the final image. The MonacoEZColour software uses this image to compare with included reference files to create and export a calibrated colour profile compatible with Photoshop. After the profile is created the scan resolution (dpi) can be changed, but all other settings should be kept constant while imaging samples.

This procedure may be repeated by scanning the channel area, the outer shell area corresponding to the placement indicia, and the combination of channel and placement indicia areas (i.e. channel overlaying the placement indicia). For convenience of handing, the sample size may be a 10 mm by 30 mm piece, however, as will be appreciated by the person skilled in the art, smaller samples sizes can be used. Precondition samples at about 23°C ± 2 C° and about 50% ± 2% relative humidity for 2 hours prior to testing.

Open the scanner lid and place the specimen onto the scanner glass with the first outer surface facing the glass. Cover the specimen with the white background (in this test method white is defined as having L* > 94, -2 < a* < 2, and -2 < b* < 2) and close the lid. Acquire and import a scan of the specimen into Photoshop at 600 dpi and 24 bit colour. Assign the calibrated scanner profile to the image and change the mode to Lab Colour ("Lab Colour" in Photoshop corresponds to the CIE L* a* b* standard). Measure and record L* a* b* values for the channel by taking 10 measurements at random locations of the image. Average the 10 individual L* a* b* values and record as L1, a1, and b1 respectively. Repeat the measure for the positioning indicia images, and record the averaged values as L2, a2 and b2. Calculate and report the colour difference (delta E*) between the bonded and unbonded areas using the following equation (the same procedure can be used for calculating delta E* between the first colour of the channel area and the third colour of the channel overlaying the positioning indicia):

$$\text{delta } E^* = \sqrt{(L_2^* - L_1^*)^2 + (a_2^* - a_1^*)^2 + (b_2^* - b_1^*)^2}$$

and report to the nearest 0.01 units. A total of three substantially identical samples are measured for each sample set. Average the three delta E** values and report to the nearest 0.1 unit.

Other colour analyses that may be useful are made using the calculations of delta Chroma (delta C*) and delta Hue (delta H*).

$$\text{Delta } C^* = \text{square-root}(a^*{}_1{}^2 + b^*{}_1{}^2) - \text{square-root}(a^*{}_2{}^2 + b^*{}_2{}^2)^2$$

$$\text{Delta } H^* = \text{square-root}[(a^*{}_2 - a^*{}_1)^2 + (b^*{}_2 - b^*{}_1)^2 - (\text{Delta} C^*)^2]$$

It is supposed that the present invention is not restricted to any form of realization described previously and that some modifications can be added to the presented embodiments without reappraisal of the appended claims.

**Claims**

1. A disposable absorbent insert (1) comprising:

   a liquid permeable topsheet (2);
   a liquid impermeable backsheet (3); and
   an absorbent core (4) comprising absorbent material therein and being sandwiched between said topsheet (2) and backsheet (3);
   wherein the insert comprises a perimeter formed by first and second transverse edges (6, 7) and first and second longitudinal edges (8, 9) connecting the first and second transverse edges (6, 7); a longitudinal centerline (y) extending substantially parallel to said first and second longitudinal edges (8, 9) and interposed therebetween such to divide said insert (1) into a first longitudinal half between said longitudinal centerline (y) and said first longitudinal edge (8) and a second longitudinal half between said longitudinal centerline (y) and said second longitudinal edge (9); and a transverse centerline (x) extending substantially parallel to said first and second transverse edges (6, 7) and interposed therebetween such to divide said insert (1) into a first transverse half between said transverse centerline (x) and said first transverse edge (6) and a second transverse half between said transverse centerline (x) and said second transverse edge (7);
   **characterised in that** the absorbent core (4) comprises a core wrap wherein a top layer (14) of the core wrap is adhered to a bottom layer (15) of the core wrap such that one or more channels (13) substantially free of absorbent material are formed, wherein said channels (13) are substantially translucent when viewed in a planar direction from the topsheet-side of said insert (1) and **in that** said insert (1) is joinable to a re-usable outer shell (20), and wherein, when joined, a portion and/or element of said outer shell (20) is viewable through said channels.

2. An insert (1) according to Claim 1 wherein the re-usable outer shell (20) comprises placement indicia (PI) having a shape that substantially corresponds to the shape of the channels (13), as viewed in a planar direction, and that may be seen through the channels (13) when positioned thereon.

3. An insert (1) according to any of the preceding Claims further comprising a wetness indicator (10), preferably wherein the wetness indicator comprises a composition that changes appearance and/or colour when contacted with urine, the composition comprising a pH indicator and/or a water-soluble dye.

4. A re-usable outer shell (20) comprising:

a front (F) and back (B) waist region having uppermost and lowermost edges; and
a crotch region (C) interposed between the front and back waist region,
wherein the uppermost edges of the waist region form a waist opening and the lowermost edges of the waist region together with lateral extremities of the crotch region (C) form two oppositely disposed leg openings; and wherein at least the body-facing surface of the crotch region (C) is adapted for receiving an absorbent insert (1) according to any of the preceding claims;
and wherein at least a portion of said crotch region (C) comprises a placement indicia (PI) having a shape, when viewed in a planar direction, that substantially corresponds to the shape of said channels (13), wherein said indicia (PI) is viewable through said channels (13) when the insert (1) is joined to outer shell (20).

5. A re-usable outer shell (20) according to Claim 4 comprising re-fastenable or permanent side seams joining the front and back waist regions, preferably the shell being in the form of a pant.

6. A re-usable outer shell (20) according to Claim 4 wherein the back waist region comprises, preferably two, transversely extending side panels (21, 21') each comprising a fastener (22, 22') for coupling to a landing zone positioned at a garment facing side of the front waist region (F), preferably the shell being in the form of a diaper.

7. A re-usable outer shell (20) according to any of Claims 4 to 6 wherein the placement indicia (PI) comprises, preferably consists of, a stiffening element having a higher resistance to bending compared to any other position and/or element of the outer shell (20) being at least proximally inboard and/or outboard of said indicia (PI).

8. A re-usable outer shell (20) according to any of Claims 4 to 7 wherein the placement indicia (PI) comprises a first graphic and/or colour that is viewable through the channels (13) when the insert (1) is joined to the outer shell (20).

9. A re-usable outer shell (20) according to any of Claims 4 to 8 wherein the placement indicia (PI) has a first colour and wherein the channels (13) have a second colour, and wherein the delta E* between the indicia (PI) and the channels (13) is at least 0.75, preferably at least 0.85, according to the method herein.

10. A re-usable outer shell (20) according to any of Claims 4 to 9 wherein placement indicia (PI) has a first colour, the channels (13) have a second colour, and wherein the channels (13) when overlaying the placement indicia (PI) have a third colour; and wherein the delta E* between the second colour and third colour is from greater than 0 to 2.0, preferably from 0.1 to 1.5, even more preferably from 0.2 to 1.0, even more preferably from 0.3 to 0.85, even more preferably from 0.4 to 0.70, according to the method herein.

11. A re-usable outer shell (20) according to any of Claims 4 to 10 wherein the crotch region (C) substantially corresponding to the position of the wetness indicator (10), when the insert (1) is joined to the shell (20), comprises: a window (23) for viewing said wetness indicator (10) from a garment-facing side of said shell (20) and/or an optical sensor (OS).

12. A re-usable outer shell (20) according to any of Claims 4 to 11 wherein the optical sensor (OS) is comprised by a detection device further comprising one or more light sources, preferably a plurality of light sources having at least a first colour and a second colour, wherein the first colour is blue and the second colour is red, preferably said light sources consisting of LEDs, more preferably said light sources being positioned at a distance from each other and from the optical sensor; even more preferably the detection device further comprising a battery and a transmitter and being detachably connected to said outer shell.

13. An absorbent assembly comprising:

an insert (1) according to any of Claims 1 to 3; and
an outer shell (20) according to any of Claims 4 to 12; preferably wherein the placement indicia (PI) has a first colour, the channels (13) have a second colour, and wherein the channels (13) overlaying the placement indicia (PI) has a third colour; and wherein the delta E* between the first colour and second colour is at least 0.70, preferably at least 0.75, according to the method herein.

14. The absorbent assembly of Claim 13 wherein the channels (13) have a first width and the placement indicia (PI) has a second width, and wherein the second with is greater than the first width, preferably wherein the second with is from 20% to 100%, preferably from 25% to 75%, even more preferably from 30% to 60%, greater than the first width.

15. A kit of parts comprising:

a plurality of inserts (1) according to any of Claims 1 to 3; and
one or more outer shells (20) according to any of Claims 4 to 12, preferably wherein the plurality of inserts (1) differ in at least one of: size, absorbency, biodegradable and/or compostable content, shape, and number of components contained therein, channel shape, channel size, and channel position.

FIG. 1A

FIG. 1B

**FIG. 2**

FIG. 3

FIG. 4

20

OS

FIG. 5

**FIG. 6**

Europäisches
Patentamt

European
Patent Office

Office européen
des brevets

# EUROPEAN SEARCH REPORT

Application Number

EP 21 21 4289

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2014/257229 A1 (WANG SAMANTHA CHEN-YEE [US] ET AL) 11 September 2014 (2014-09-11) * paragraphs [0008], [0009], [0182] – [0301]; claims; figures * ----- | 1-15 | INV. A61F13/49 A61F13/15 A61F13/505 |
| Y | EP 2 886 093 A1 (PROCTER & GAMBLE [US]) 24 June 2015 (2015-06-24) * paragraphs [0051] – [0117]; claims; figures * ----- | 1-15 | |

TECHNICAL FIELDS
SEARCHED       (IPC)

A61F

The present search report has been drawn up for all claims

| Place of search | Date of completion of the search | Examiner |
|---|---|---|
| Munich | 20 May 2022 | Boccignone, Magda |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
..................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 21 4289

This annex lists the patent family members relating to the patent documents cited in the above-mentioned European search report.
The members are as contained in the European Patent Office EDP file on
The European Patent Office is in no way liable for these particulars which are merely given for the purpose of information.

20-05-2022

| Patent document cited in search report | | | Publication date | Patent family member(s) | | Publication date |
|---|---|---|---|---|---|---|
| US 2014257229 | A1 | | 11-09-2014 | CN | 105007876 A | 28-10-2015 |
| | | | | EP | 2964176 A1 | 13-01-2016 |
| | | | | JP | 2016512060 A | 25-04-2016 |
| | | | | US | 2014257229 A1 | 11-09-2014 |
| | | | | WO | 2014138274 A1 | 12-09-2014 |
| EP 2886093 | A1 | | 24-06-2015 | CN | 105828766 A | 03-08-2016 |
| | | | | EP | 2886093 A1 | 24-06-2015 |
| | | | | JP | 6258495 B2 | 10-01-2018 |
| | | | | JP | 2017500096 A | 05-01-2017 |
| | | | | US | 2015173967 A1 | 25-06-2015 |
| | | | | WO | 2015094732 A1 | 25-06-2015 |

EPO FORM P0459

For more details about this annex : see Official Journal of the European Patent Office, No. 12/82

## REFERENCES CITED IN THE DESCRIPTION

**Patent documents cited in the description**

- EP 3842017 A1
- EP 3659563 A1
- EP 3895673 A1
- EP 3888606 A1
- US 20200054782 A1
- US 4414398 A
- EP 21152698